# EUROPEAN PATENT SPECIFICATION

(11) **EP 3 435 928 B1**
(45) Date of publication and mention of the grant of the patent: **13.09.2023**
(21) Application number: 16726419.1
(22) Date of filing: 31.03.2016
(51) Int. Cl.: A61F 2/88, A61F 2/915

(54) **HELICAL ULTRA LOW FORESHORTENING STENT**
SPIRALFÖRMIGER STENT MIT ULTRAGERINGER VERKÜRZUNG
ENDOPROTHÈSE HÉLICOÏDALE CARACTÉRISÉE PAR UN RACCOURCISSEMENT EXTRÊMEMENT FAIBLE

(43) Date of publication of application: 06.02.2019
(73) Proprietor: Cordis US Corp., Miami Lakes, FL 33014 (US)
(72) Inventor: MAJERCAK, David, Livermore, CA 94550 (US)
(74) Representative: Marks & Clerk LLP
(86) International application number: PCT/IB2016/000597
(87) International publication number: WO 2017/168196

(56) References cited:
- WO-A1-98/38945
- WO-A1-2016/060833
- US-A- 5 810 872
- US-A1- 2002 095 206
- US-A1- 2006 079 955
- US-A1- 2007 005 126
- US-A1- 2010 094 394

## Description

### BACKGROUND

It is well known to employ various intravascular endoprostheses delivered percutaneously for the treatment of diseases of various body vessels. These types of endoprosthesis are commonly referred to as "stents". A stent (which includes covered stents) is a generally formed longitudinal tubular device of biocompatible material, such as stainless steel, cobalt-chromium, nitinol or biodegradable materials, having holes or slots cut therein so they can be radially expanded, by a balloon catheter or the like, or alternately selfexpanded due to its shape memory characteristic within a biological vessel. The stents are usually configured as a series of hoops with each defined by cylindrical-like framework. The framework is usually a series of alternating sequence of struts with a vertex between each pair of struts and configured so that the vertex of one hoop facing a vertex of the adjacent hoops may be connected together.

Stents are useful in the treatment of cardiac arterial diseases, stenosis, strictures or aneurysms in biological vessels such as blood vessels. These devices are implanted within the vessel to reinforce a collapsing, partially occluded, weakened or abnormally dilated sections of a vessel. Stents are typically employed after angioplasty of a blood vessel to prevent restenosis of the diseased vessel. While stents are most notably used in blood vessels, stents may also be implanted in other body vessels such as the urogenital tract and bile duct.

Stents (and stent-graft) generally include an open flexible configuration. This configuration allows the stent to be inserted through curved vessels. Furthermore, the stent configuration allows the stent to be configured in a radially compressed state for intraluminal catheter implantation. Once properly positioned adjacent the damaged vessel, the stent is radially expanded so as to support and reinforce the vessel. Radial expansion of the stent can be accomplished by inflation of a balloon attached to the catheter, or alternatively using self-expanding materials such as nitinol within the stent. Examples of various stent constructions are shown in U.S. Pat. No. 4,733,665 filed by Palmaz on Nov. 7, 1985. Stents that utilize a helical configuration (as opposed to a series of right cylindrical hoops) have also been utilized such as, for example, shown and described in EP964658;
EP1229864; EP1637090; EP2049189; US7988723; US5931866; US6132461; US6432132; US6551350; US7108714; US7670367;US7682380; US7942922; US7967852; US7967852; US8382820; US8460364;US8696733; US8834557; US8961590; US20010007955; US20020002400; US20020099436; US20040193246; US20060064155; US20060074480; US20070150045; US20080051874; US20080140173; US20080195189; US20080195196; US20100087906; US20100145434; US20100324661; US20100324662; US20120029621; US20120029622; US20130131782; US20130238084; US20140081382; US20140088683; US20140336748; US20140379074; US5931866; US6551350; US7108714; US7670367; US7682380; US7942922; US7967852; US8382820 ; US8460364; US8696733; US8834557; US8961590; US8968385; WO1998027893; WO2000066034; WO2001035864; WO2004091381; WO2006026781; WO2006133960 ; WO2008011614; WO2008021006; WO2008027188; WO2008098926; WO2008098927; WO2012018840; WO2012018844; or WO2013114214.
US 2006/079955 A1 discloses a stent comprising a helically wound ribbon of material comprising a plurality of ribbon turns about a longitudinal axis of the stent. US 2010/094394 A1 discloses a self-expanding stent combining a helical strut band helically wound about an axis of the strut and interconnected by coil elements helically wound about an axis of the stent and progress in the same direction as the helical strut band.

As known in the art, there are generally two main types of stent configurations. A first type, referenced as "fully connected stents," may have all of the vertices on one hoop connected to all of the vertices on adjacent hoops. It is noted that a fully connected stent does not have to have its vertices connected to vertices as long as each pair of struts in one framework is connected to another pair of struts on adjacent frameworks. A second type is referred to as "partially connected stents" which may not have all of the vertices on one hoop connected to all of the vertices on adjacent hoops. The partially connected stents have the benefits of flexibility at the cost of better
scaffolding or the surface area of the stent being applied against the inner surface of the body vessel. The fully connected stents have the advantages of allowing the stent to be recaptured back into a delivery catheter as well as increasing the surface area in which the stent framework is applied to the inner surface of the body vessel. One problem with fully connected stents is that a length of the stent in an "uncrimped" or deployed condition is much shorter than the length of the stent in the "crimped" or "compressed" condition. This long standing problem is known in the field as "stent foreshortening." Stents, particularly those that are fully connected from one framework to the adjacent frameworks, can foreshorten after diametrical expansion by as much as 30% to 50% of its crimped (undeployed or unexpanded diameter) length. In a typical interventional procedure, a surgeon may move the crimped (undeployed) stent (inside a delivery sheath catheter) towards a target lesion in a body vessel (e.g., artery, vein, ducts etc). Believing that the undeployed stent (in the catheter sheath) will expand against the target lesion, the surgeon retracts the delivery sheath, thereby allowing the stent to expand against the lesion. But because of the "foreshortening" characteristics of the known stent, such stent may not actually deploy at the target lesion due to the stent becoming shortened after diametric expansion. This could lead to the surgeon having to deploy multiple stents or to use unnecessarily long stent to ensure that the target lesion is not missed.

### SUMMARY OF THE DISCLOSURE

Applicant has devised a new implantable prosthesis (e.g., stent or stent-graft) that virtually eliminates the foreshortening effects of such prosthesis. The present invention relates to an implantable prosthesis comprising:
a. a first framework circumscribing in a first helical path about a longitudinal axis (L-L) proximate a first end to proximate a second end of the prosthesis, the first framework including a plurality of struts disposed in a first sinusoidal pattern along the first helical path with a primary apex portion connected for every pair of struts;
b. a second framework spaced from the first framework, the second framework circumscribing a second helical path about the longitudinal axis (L-L) proximate the first end to proximate the second end, the second framework including a plurality of struts disposed in a second sinusoidal pattern along the second helical path with a secondary apex portion connected to a pair of struts for every pair of struts; and
c. a plurality of connectors, each connector is connected to proximate a primary apex portion of the first framework and to proximate a secondary apex portion of the secondary framework,
wherein the first helical path and second helical path are interleafed; and, each primary apex portion connected to a secondary apex portion on an adjacent framework and wherein each primary apex portion is one vertex offset circumferentially from each corresponding secondary apex portion

For each of the embodiments described above, the following features can be utilized in various permutations with each of the embodiments. For example, each of the plurality of connectors may include a linear member; the adjacent connectors may be configured in a generally parallel configuration; the first connector portion connects a vertex of the first framework to a vertex of the second frame work
circumferentially offset by one vertex of the second framework; the stent may include a first length when crimped or compressed to have a smaller outer diameter and a second length of no less than about 90% of the first length when the stent is uncompressed to expand to a larger outer diameter; the first framework may define a first helical angle with respect to the longitudinal axis and the second framework may define a second helical angle with respect to the longitudinal axis when the stent is in an uncompressed configuration; the connector portions may define a third helical angle with respect to the longitudinal axis when the stent is in an uncompressed configuration; the first helical angle may generally be equal to the second helical angle; the third helical angle may be different from the first helical angle or the second helical angle; the third helical angle may be less than either of the first helical angle or the second helical angle; the first helical angle may be about 65 degrees with respect to the longitudinal axis; the second helical angle may be about 65 degrees with respect to the longitudinal axis; the third helical angle may be about 30 degrees with respect to the longitudinal axis; the stent may include a first length when crimped or compressed to have a smaller outer diameter and a second length of no less than about 90% of the first length when the stent is uncompressed to expand to a larger outer diameter; the first framework may have a first starting location generally diametrically opposed to a second starting location of the second framework as referenced with the longitudinal axis; the stent may include a biocompatible metal or alloys of metal or metals; the stent may include a biocompatible polymer; the stent may include a combination of biocompatible metal and polymer; the stent may include a drug coating disposed on surfaces of the stent.

The expandable frame may be one of a self-expanding frame or a balloon expandable frame which frame can be of at least a bioresorbable material. The frame may include a series of hoops connected to each other via connectors of the same material as the frame. Alternatively, the frame may include a series of hoops independent from each other so that the hoops are connected indirectly through the graft material. The graft materials may be composed of various polymeric formulations includes PET (polyester), Fluoro-polymers such as PTFE and FEP, spun PTFE, and HDPE.

In the case of a graft where no internal or external frame is needed, a thin-film graft made from nitinol can be utilized. The "thin-film" material for the graft can be made from well-known chemical deposition or physical deposition techniques. Chemical deposition can be by plating, chemical solution deposition, spin coating, chemical vapor deposition, plasma enhanced vapor deposition, or atomic layer deposition. Physical deposition for thin film manufacturing can be by thermal evaporator, laser deposition, cathodic arc deposition, sputtering, vapor deposition, ion-beam assisted evaporative deposition or electrospray deposition.

These and other embodiments, features and advantages will become apparent to those skilled in the art when taken with reference to the following more detailed description of the exemplary embodiments of the invention in conjunction with the accompanying drawings that are first briefly described. As well, it is intended that these embodiments, features and advantages may be claimed in applications for patents.

### BRIEF DESCRIPTION OF DRAWINGS

The accompanying drawings, which are incorporated herein and constitute part of this specification, illustrate presently preferred embodiments of the invention, and, together with the general description given above and the detailed description given below, serve to explain features of the invention (wherein like numerals represent like elements), in which:
FIG. 1 illustrates a perspective view of one embodiment of the prosthesis in accordance with the present invention;
FIG. 2A illustrates a side view of the prosthesis shown in Fig. 1;
FIG. 2B illustrates a side view of a known prosthesis for comparison with the prosthesis of FIG. 2A;
FIG. 3A illustrates a magnified side view of the prosthesis of Fig. 2A;
FIG. 3B illustrates a magnified side view of the known prosthesis of Fig. 2B for comparison purposes;
FIG. 4 illustrates the prosthesis of Fig. 1, in a crimped (compressed) configuration, which prosthesis has been cut so that it can be laid flat on a planar surface;

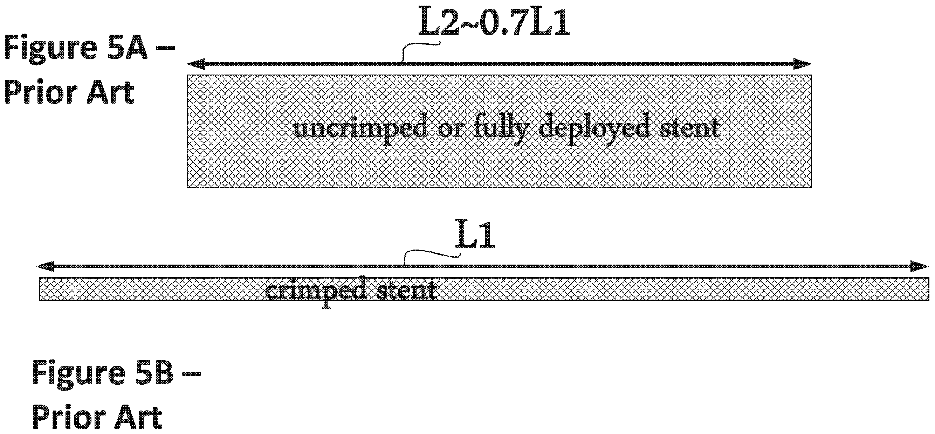

FIGS.5A and 5B illustrate the "foreshortening" effect in known implantable prosthesis;
FIG. 6 is a screenshot illustrating the ultra-low foreshortening effect of one embodiment made by applicant.

### MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment. The term "stent" is intended to encompass an uncovered framework as well as one that is covered by a suitable material (e.g., stent-graft).

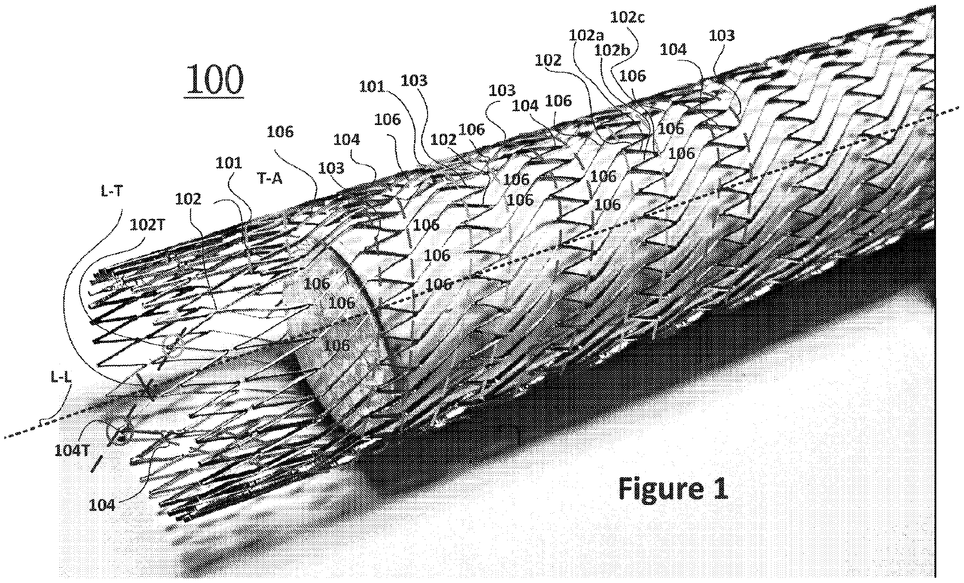

Referring now to the figures wherein like numerals indicate the same element throughout the views, there is shown in FIG. 1 an implantable prosthesis 100 in an uncrimped and fully expanded configuration. For ease of viewing the structures, implantable prosthesis 100 has been mounted on a white cylinder to prevent the rear cylindrical structures from interfering with an inspection of the front cylindrical structure. As shown, the exemplary implantable prosthesis 100 has two helical frameworks 102 and 104 adjacent to one another to present what appears to be a "double helix" configuration but in reality should be considered as a "tri-helix" stent structure. It should be noted that while a tri-helix is described and illustrated the scope of the claimed invention is intended to cover variations of more than three helical stent structures. And as used herein, the term "framework" includes discrete segments of the stent connected (e.g., welded or bonded) together to define a frame and would also include a framework made by cutting out unneeded portions from a rod stock (e.g., hollow, circular or polygonal) such that an integral framework without any substantial discontinuities (caused by e.g., brazing or welding) is obtained.

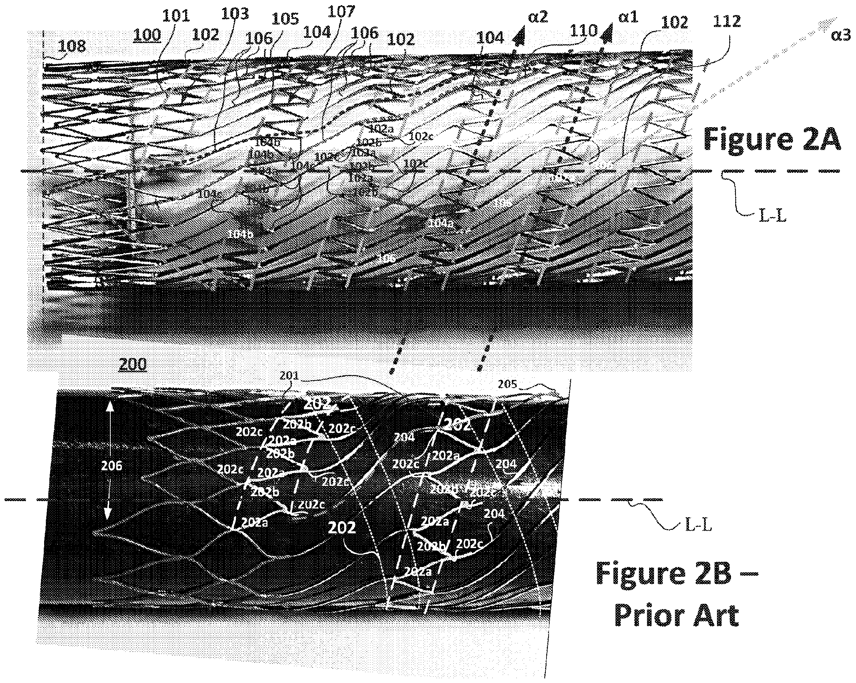

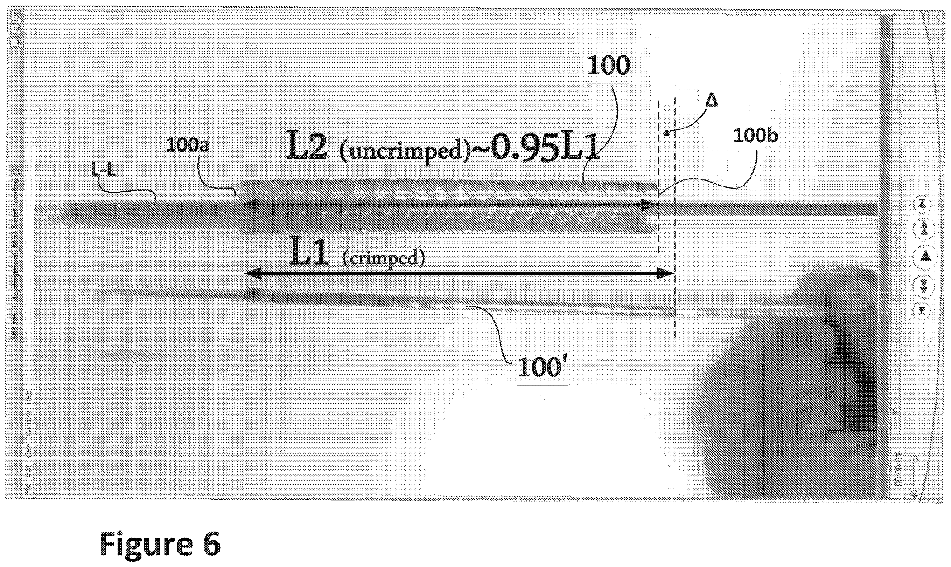

In the tri-helix configuration, as devised by applicant as one of many embodiments available, allows for the heretofore novel ultra-low (i.e., less than 20% and actually 5% or less) foreshortening characteristic of the prosthesis (e.g., stent) noted in my summary above as well as other configurations that allows the length of the stent or prosthesis to be longer (as opposed to being shorter) when the stent is uncompressed as compared to the length of the same stent in its crimped or compressed configuration. In particular, the implantable prosthesis 100 includes a first framework 102 that circumscribes a longitudinal axis L-L along a first helical path 101 from a first end 100a to a second end 100b (Fig. 6). The first framework 102 includes a plurality of
struts 102a and 102b (Fig. 2A) disposed in a first sinusoidal pattern 105 along the first helical path 101. As used herein, the term "sinusoidal" is not intended to limit the implantable prosthesis configuration to a pure sinusoidal pattern. That is, the term "sinusoidal" includes any repeating sequence (e.g., zig-zag) of two struts (linear or curvilinear segments) connected to a vertex to define a stent framework such that adjacent vertices of one framework are spaced back and forth along a longitudinal axis while circumscribing on a virtual inside radius about the longitudinal axis.

The framework 102 includes a primary apex portion 102c connected to a pair of struts 102a, 102b to define the first sinusoidal pattern 103. The second framework 104 is spaced from the first framework 102, similar to the double helix. In particular, the second framework 104 circumscribes a second helical path 105 about the longitudinal axis L-L proximate the first end 100a to proximate the second end 100b. Similar to the first framework 102, the second framework 104 includes a plurality of struts 104a, 104b disposed in a second sinusoidal pattern 107 along the second helical path 103 with a secondary apex portion 104c connected to a pair of struts 104a, 104b to define the second sinusoidal pattern 107.

Although the strut pairs shown are generally identical, each of the strut pairs can be of a different configuration. In circumscribing and translating along the axis L-L, the helical path 102 or 104 follows a portion of a complete circle, except near the terminal ends of the stent, while at the same time translating along the axis L-L. As such, a plurality of arcuate sections defined by the successive pairing of strut pairs with a vertex connected between the strut pairs circumscribes the axis L-L from a terminal first end to a terminal second end of the helical path to thereby define a partially cylindrical shape. Further, although the arcuate sections are shown as being formed by alternating strut pairs, similar designs may employ struts that are disposed in an
undulating, sinusoidal or wave-like pattern.

To aid the viewer in visualization of the helical path about the axis L-L as defined by the zig-zag struts and vertices (i.e., 102a-c and 104a-c), the helical path is illustrated using dashed lines generally circumscribing about the axis L-L with the prosthesis 100 having a first end 100a and a second end 100b (Fig. 6). To further aid visualization, stent 100 is illustrated with only the foreground structure, with the background structure such as struts that continue the helical path being hidden. It is
noted that where the application of a covered stent is desired, the helical path is also a representation of another embodiment where the struts are covered (partially or wholly) by a suitable material (e.g., ePTFE, Dacron, Nylon, fibrin, to name a few).

To ensure that the implantable prosthesis functions for its intended purpose, a plurality of connectors 106 is provided to connect one helix (e.g., 102) to the adjacent helix (e.g., 104) and from the second helix to the first helix and repeating in such sequence. In particular, each connector 106 is connected to proximate a primary apex portion 102c of the first framework 102 and a secondary apex portion 104c of the secondary framework 104. This arrangement is repeated for at least one helix (102 or 104) to the other helix (104 or 102). Each vertex of one helix is connected to a vertex on the adjacent helix. By this arrangement of a connector 106 to a strut 104a to a strut 106 to a strut 102a to a strut 106 and so on, a third helix 110 is formed, as can be seen in Fig. 2A.

Referring to Fig. 2B of the known prior art helical implantable prosthesis 200, it can be seen that framework 201 (with attendant struts 202a, 202b and vertex 202c) defines a first helical path 202 while connectors 204 connects vertices 202c of the same helix together to define a second helical arrangement 205. It has been observed that this implantable prosthesis configuration may have as much as 30% or more of the shortening (or foreshortening) of its length (in the crimped configuration) when deployed. That is, the axial length of the stent 200 in the deployed uncompressed configuration (with a larger outside diameter) is shorter than the length of the stent 200 in its compressed or crimped configuration (with a smaller outside diameter).

Referring back to Fig. 2A, it is noted that each primary apex portion 102c is connected via the connector 106 to each secondary apex portion 104c while circumferentially offset ( on a virtual radius about axis L-L) to such primary apex portion 102c so that the connector 106 defines a helix angle a3 with respect to the longitudinal axis L-L.

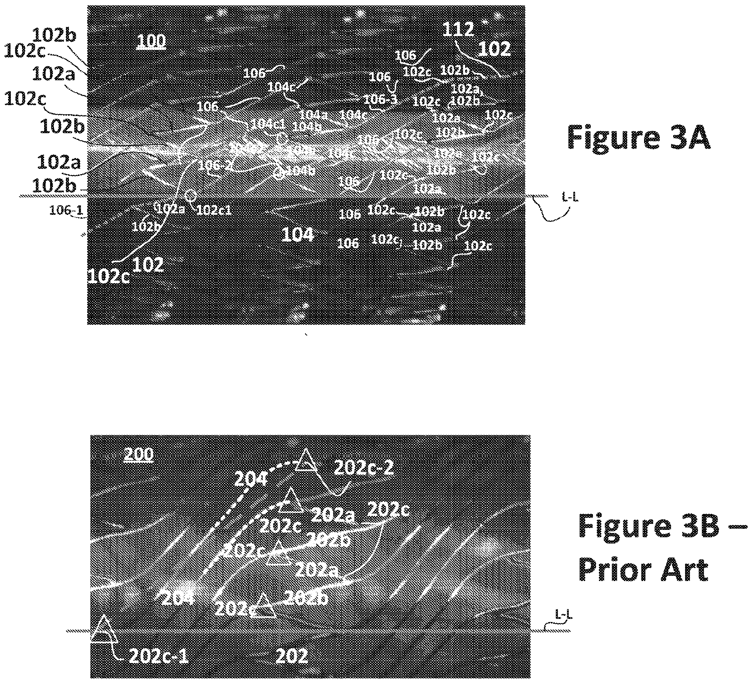

In the preferred embodiments, shown in Fig. 3A, the primary apex portion 102c1 may be connected via the connector 106-2 to each secondary apex portion 104c1 (upper right circle) but circumferentially offset (with respect to the longitudinal axis L-L) to such primary apex portion 102c1 by one secondary apex portion 104c2. In other words, the first connector portion 106 connects at least one vertex 102c1 of the first
framework 102 to at least one vertex 104c1 of the second framework 104 but the vertex of the second framework 104 is not vertex 104c2 directly facing vertex 102c1 but is the vertex 104c1 which is offset circumferentially from vertex 102c1 of the first framework 102. This is in contrast to the known stent 200, shown here in Fig. 3B, in which the connector 204 connects vertex 202c-1 to vertex 202c-2 which is three vertices offset circumferentially from vertex 202c-1.

Other differences can be seen between Figs. 3A and 3B. In Fig. 3A, a second connector portion 106 connects at least one other vertex of the second framework 104 to at least one other vertex of the first framework 102 along the axis L-L to thereby define a third helix 112 with respect to the longitudinal axis L-L. It is noted that each of the plurality of connectors may be a linear member but it is within the intended scope of the claims that the connectors can be configured as curved members or a combination of linear and curved members ("curvilinear"). In the preferred
embodiment, adjacent connectors 106 are disposed in a generally parallel configuration from the first framework 102 to the second framework 104, shown here in Fig. 3A. In contrast, the connectors 204 are not parallel to each other proximate the vertices 202c.

Referring back to Fig. 2A, it can be seen that the first framework defines a first helical angle α1 with respect to the longitudinal axis L-L and the second framework 104 defines a second helical angle α2 with respect to the longitudinal axis L-L when the implantable prosthesis is in an uncompressed configuration as shown here. Similarly, the connector portions 106 define a third helical angle α3 with respect to the longitudinal axis L-L in the uncrimped or fully opened operational state of implantable prosthesis 100. The helical angle is measured by superimposing of the subject component against the longitudinal axis on a plane or planar surface. In the preferred embodiments, the first helical angle α1 may be configured to be generally equal to the second helical angle α2 and the third helical angle α3 can be configured to have a different magnitude from the first helical angle or the second helical angle. In particular, the third helical angle is less than either of the first helical angle α1 or the second helical angle α2. In one prototype, the first or the second helical angle is about 65 degrees with respect to the longitudinal axis and the third helical angle is about 30 degrees with respect to the longitudinal axis. This relationship of the third helical angle being half of the first or the second helical angles is believed to allow for the ultralow foreshortening characteristic of my invention.

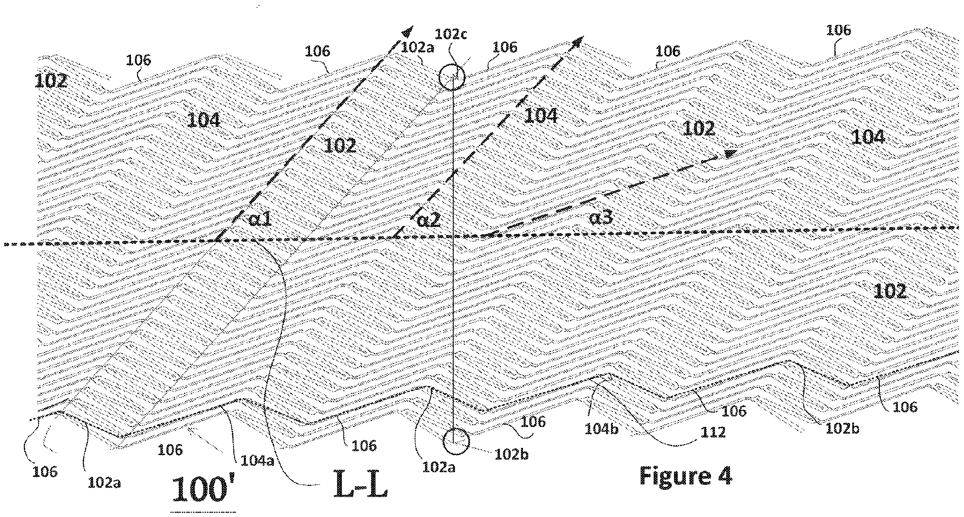

Referring to Fig. 4, the exemplary implantable prosthesis 100 is illustrated in its crimped state but unrolled from its cylindrical configuration (of Fig. 1) so that the three helix can be viewed as a whole with respective helical angles α1 (for first framework 102), α2 (for second framework 104), and α3 (for the third helical framework 112, as defined by the combination of connector 106, struts 102a, 104a, 106, 102a, 106, 104b, 106, 102a, 106 and so on as shown here in dashed lines). In this flattened configuration, it can be seen that a first framework 102 has a strut 102a connected to a vertex or apex 102c (top of Fig. 4). This vertex 102c is connected to strut 102b while bypassing the second framework 104. By devising the various components of implantable prosthesis 100 in this manner, applicant was able to achieve an ultra-low foreshortening performance of the exemplary implantable prosthesis, as shown in Fig. 6.

Although the combination of helical angles α1, α2, and α3 have been devised for this exemplary embodiment, other combination of first helical angle, second helical angle and third helical angle can be devised for different magnitudes of foreshortening.

Figure 6 is a screen shot of a video made to illustrate the many performance improvements of my design. In particular, two identical implantable prosthesis 100 were used in different operational states (crimped and uncrimped). To indicate their different operational states, the uncrimped implantable prosthesis (with a larger outside diameter OD2) is referenced as 100 while the crimped (compressed and retained in a catheter sheath to a smaller outside diameter OD1) is referenced as 100'. As can be seen visually (and measured with a suitable instrument) the length L2 of the uncrimped implantable prosthesis 100 is at least 80% (and actually 95%) of the crimped implantable prosthesis length Ll. This reduction in the foreshortening characteristic for a "fully connected" implantable prosthesis (i.e., a stent in which all vertices of one framework (or hoop) are connected to adjacent vertices of the adjacent frameworks or hoops) is believed to be the first of its kind to resolve this long standing problem that has been prevalent in this field.

Certain other features have been devised to ensure that any force applied to the ends of the implantable prosthesis tends to be evenly distributed across the framework.

In particular, as shown in Fig. 1, the first and second helical frameworks are configured so that they begin at generally diametrically opposed positions 102T and 104T where a line L-T intersects the longitudinal axis and the two starting positions 102T and 104T of the respective first and second helical frameworks. By placing the two starting locations of the two helical frameworks at diametrically opposed location, exemplary stent 100 resolves a problem of uneven stent framework distribution in a helical type stent. This uneven stent framework distribution can be seen in the known helical stent 200 in which there is a gap 206 at either of the ends of the stent 200. This gap 206 of the known stent 200 is believed to cause uneven loading on the rest of the framework for stent 200 which could affect how the stent 200 is deployed, including its propensity for "stent jumping" during sheath retraction and deployment of stent 200. Another benefit of my design is the provision of hoops with diamond-like stent framework of different sizes disposed proximate the terminal ends to allow the terminal end 108 of the stent 100 to approximate a cylindrical profile while matching to this terminal end 108 profile to the helical stent frameworks, seen here in Figs 1 and 2A. The varying size of the diamond framework allows the stent remains symmetrical during deployment as a catheter sheath is retracted due to the even loading of the terminal end 108 against a stationary rod inside the catheter with a profile similar to terminal end 108. Additionally, the squared end 108 of the stent is preferred by physicians as this configuration allows to determine what part of the body vessel is covered by the stent. As well, this allows for additional stents to stack up tightly against each other in the body vessel.

It is noted that stent 100 can be cut from a tube. Thereafter, stent 100 can be expanded in the duct or vessel of a host by a separate mechanism (e.g., balloon) or by utilization of a material that self-expands upon predetermined implantation conditions, e.g., body temperature. The stent 100 can be formed from a suitable biocompatible material such as, for example, metal, metal alloys, shape memory materials, polymer metals and other biocompatible materials which may be bioabsorble. Preferably, the prosthesis are laser cut from small diameter tubing from biocompatible metals such as shape memory materials or balloon expandable materials. Details of this particular embodiment of the stent can be gleaned from US Patent 8,328,864.

The implantable prosthesis 100 can be covered on its outside surface (the surface contacting the body vessel), on the inside surface or both inner and outer surfaces with a suitable graft material. Graft material for prosthetic 100 can be made from a suitable material such as, for example, PTFE, ePTFE, Dacron, PET (polyester), Fluoro-polymers such as PTFE and FEP, spun PTFE, HDPE, and combinations thereof. Either or both of the graft and implantable prosthesis can be formed from
biodegradable polymers such as polylactic acid (i.e., PLA), polyglycolic acid (i.e., PGA), polydioxanone (i.e., PDS), polyhydroxybutyrate (i.e., PHB), polyhydroxyvalerate (i.e., PHV), and copolymers or a combination of PHB and PHV (available commercially as Biopol^{®}), polycaprolactone (available as Capronor^{®}), polyanhydrides (aliphatic polyanhydrides in the back bone or side chains or aromatic polyanhydrides with benzene in the side chain), polyorthoesters, polyaminoacids (e.g., poly-L-lysine, polyglutamic acid), pseudo- polyaminoacids (e.g., with back bone of polyaminoacids altered), polycyanocrylates, or polyphosphazenes. As used herein, the term "bio-reabsorbable" includes a suitable biocompatible material, mixture of materials or partial components of materials being degraded into other generally non-toxic materials by an agent present in biological tissue (i.e., being biodegradable via a suitable mechanism, such as, for example, hydrolysis) or being removed by cellular activity (i.e., bioresorption, bioabsorption, or bioresorbable), by bulk or surface degradation (i.e., bioerosion such as, for example, by utilizing a water insoluble polymer that is soluble in water upon contact with biological tissue or fluid), or a combination of one or more of the bio-degradable, bio-erodable, or bioresorbable material noted above.

In certain applications where a fabric or a polymeric material is not desired, the covering material (e.g., graft) can be formed by a suitable thin-film deposition technique over a substrate such as an expandable frame (self-expanding or balloon expandable implantable prosthesis). In this configuration with the thin-film, the expandable frame can be disposed on the outside surface of the thin-film (acting as a graft); the expandable frame can be sandwiched between two thin-film graft materials; or two expandable frames can sandwich the thin-film graft material.

In one embodiment, bio-active agents can be added to the polymer, the metal alloy of the frame or the thin-film for delivery to the host's vessel or duct. The bio-active agents may also be used to coat the entire graft, the entire implantable prosthesis or only a portion of either. A coating may include one or more non-genetic therapeutic agents, genetic materials and cells and combinations thereof as well as other polymeric coatings. Non-genetic therapeutic agents include anti-thrombogenic agents such as heparin, heparin derivatives, urokinase, and PPack (dextrophenylalanine proline arginine chloromethylketone); antiproliferative agents such as enoxaprin, angiopeptin, or monoclonal antibodies capable of blocking smooth muscle cell proliferation, hirudin, and acetylsalicylic acid; anti-inflammatory agents such as dexamethasone, prednisolone, corticosterone, budesonide, estrogen, sulfasalazine, and mesalamine; antineoplastic/antiproliferative/anti- miotic agents such as paclitaxel, 5-fluorouracil, cisplatin, vinblastine, vincristine, epothilones, endostatin, angiostatin and thymidine kinase inhibitors; anesthetic agents such as lidocaine, bupivacaine, and ropivacaine; anti-coagulants, an RGD peptide-containing compound, heparin, antithrombin compounds, platelet receptor antagonists, anti-thrombin anticodies, anti-platelet receptor antibodies, aspirin, prostaglandin inhibitors, platelet inhibitors and tick antiplatelet peptides; vascular cell growth promotors such as growth factor inhibitors, growth factor receptor antagonists, transcriptional activators, and translational promotors; vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; cholesterol-lowering agents; vasodilating agents; and agents which interfere with endogenous vasoactive mechanisms.

Genetic materials include anti-sense DNA and RNA, DNA coding for, anti-sense RNA, tRNA or rRNA to replace defective or deficient endogenous molecules, angiogenic factors including growth factors such as acidic and basic fibroblast growth factors, vascular endothelial growth factor, epidermal growth factor, transforming growth factor alpha and beta, platelet-derived endothelial growth factor, platelet-derived growth factor, tumor necrosis factor alpha, hepatocyte growth factor and insulin like growth factor, cell cycle inhibitors including CD inhibitors, thymidine kinase ("TK") and other agents useful for interfering with cell proliferation the family of bone morphogenic proteins ("BMPs"), BMP- 2, BMP-3, BMP-4, BMP-5, BMP-6 (Vgr-1), BMP-7 (OP-I), BMP-8, BMP-9, BMP-IO, BMP-I, BMP-12, BMP-13, BMP-14, BMP-15, and BMP-16. Desirable BMPs are any of BMP-2, BMP-3, BMP-4, BMP-5, BMP-6 and BMP-7. These dimeric proteins can be provided as homodimers, heterodimers, or combinations thereof, alone or together with other molecules. Alternatively or, in addition, molecules capable of inducing an upstream or downstream effect of a BMP can be provided. Such molecules include any of the "hedgehog" proteins, or the DNA encoding them.

Cells can be of human origin (autologous or allogeneic) or from an animal source (xenogeneic), genetically engineered if desired to deliver proteins of interest at the deployment site. The cells may be provided in a delivery media. The delivery media may be formulated as needed to maintain cell function and viability.

Suitable polymer coating materials include polycarboxylic acids, cellulosic polymers, including cellulose acetate and cellulose nitrate, gelatin, polyvinylpyrrolidone, cross-linked polyvinylpyrrolidone, polyanhydrides including maleic anhydride polymers, polyamides, polyvinyl alcohols, copolymers of vinyl monomers such as EVA, polyvinyl ethers, polyvinyl aromatics, polyethylene oxides, glycosaminoglycans, polysaccharides, polyesters including polyethylene terephthalate, polyacrylamides, polyethers, polyether sulfone, polycarbonate, polyalkylenes including polypropylene, polyethylene and high molecular weight polyethylene, halogenated polyalkylenes including polytetrafluoroethylene, polyurethanes, polyorthoesters, proteins, polypeptides, silicones, siloxane polymers, polylactic acid, polyglycolic acid, polycaprolactone, polyhydroxybutyrate valerate and blends and copolymers thereof, coatings from polymer dispersions such as polyurethane dispersions (for example, BAYHDROL^{®} fibrin, collagen and derivatives thereof, polysaccharides such as celluloses, starches, dextrans, alginates and derivatives, hyaluronic acid, squalene emulsions. Polyacrylic acid, available as HYDROPLUS^{®} (Boston Scientific Corporation, Natick, Mass.), and described in U.S. Pat. No. 5,091,205 is particularly desirable. Even more desirable is a copolymer of polylactic acid and polycaprolactone. Suitable coverings include nylon, collagen, PTFE and expanded PTFE, polyethylene terephthalate and KEVLAR^{®}, ultra-high molecular weight polyethylene, or any of the materials
disclosed in U.S. Pat. No. 5,824,046 and U.S. Pat. No. 5,755,770. More generally, any known graft material may be used including synthetic polymers such as polyethylene, polypropylene, polyurethane, polyglycolic acid, polyesters, polyamides, their mixtures, blends and copolymers.

While the invention has been described in terms of particular variations and illustrative figures, those of ordinary skill in the art will recognize that the invention is not limited to the variations or figures described. In addition, where methods and steps described above indicate certain events occurring in certain order, it is intended that certain steps do not have to be performed in the order described but in any order as long as the steps allow the embodiments to function for their intended purposes.

## Claims

1. An implantable prosthesis (100) comprising:
a. a first framework (102) circumscribing in a first helical path (101) about a longitudinal axis (L-L) proximate a first end (100a) to proximate a second end (100b) of the prosthesis, the first framework including a plurality of struts (102a, 102 b) disposed in a first sinusoidal pattern (103) along the first helical path (101) with a primary apex portion (102c) connected for every pair of struts (102a, 102 b);
b. a second framework (104) spaced from the first framework (102), the second framework circumscribing a second helical path (105) about the longitudinal axis (L-L) proximate the first end (100a) to proximate the second end (100b), the second framework (104) including a plurality of struts (104a, 104b) disposed in a second sinusoidal pattern (107) along the second helical path (105) with a secondary apex portion (104c) connected to a pair of struts for every pair of struts (104a, 104b); and
c. a plurality of connectors (106), each connector (106) is connected to proximate a primary apex portion (102c) of the first framework (102) and to proximate a secondary apex portion (104c) of the secondary framework (104),
wherein the first helical path (101) and second helical path (105) are interleafed; and, each primary apex portion (102c) connected to a secondary apex portion (104c) on an adjacent framework and wherein each primary apex portion (102c) is one vertex offset circumferentially from each corresponding secondary apex portion (104c).

2. The implantable prosthesis (100) of claim 1 in which each of the plurality of connectors (106) comprises a linear member, optionally in which adjacent connectors (106) are disposed in a generally parallel configuration.

3. The implantable prosthesis (100) of claim 1, in which the implantable prosthesis (100) includes a first length (L1) when crimped or compressed to have a smaller outer diameter and a second length of no less than about 80% of the first length when the implantable prosthesis (100) is uncompressed to expand to a larger outer diameter.

4. The implantable prosthesis (100) according to any one of the preceding claims, in which the first framework defines a first helical angle α1 with respect to the longitudinal axis (L-L) and the second framework defines a second helical angle (α2) with respect to the longitudinal axis (L-L) when the implantable prosthesis (100) is in an uncompressed configuration.

5. The implantable prosthesis (100) according to any one of the preceding claims, in which the connectors define a third helical angle (α3) with respect to the longitudinal axis (L-L) when the implantable prosthesis (100) is in an uncompressed configuration.

6. The implantable prosthesis (100) according to any one of the preceding claims, in which the first helical angle (α1) is generally equal to the second helical angle (α2).

7. The implantable prosthesis (100) of claim 5 or 6, in which the third helical angle α3 is different from the first helical angle (α1) or the second helical angle (α2);
optionally, in which the third helical angle (α3) is less than either of the first helical angle (α1) or the second helical angle (α2).

8. The implantable prosthesis (100) according to any one of the preceding claims, in which the first helical angle (α1) is about 65 degrees with respect to the longitudinal axis (L-L); and/or,
in which the second helical angle (α2) is about 65 degrees with respect to the longitudinal axis (L-L).

9. The implantable prosthesis (100) according to any one of claims 5 to 8, in which the third helical angle (α3) is about 30 degrees with respect to the longitudinal axis (L-L); and/or,
in which the third helical angle (α3) is about half of one of the first and second helical angles (α1, α2).

10. The implantable prosthesis (100) according to any one of the preceding claims, in which the implantable prosthesis (100) includes a first length when crimped or compressed (L1) to have a smaller outer diameter and a second length of no less than about 90% of the first length when the implantable prosthesis is uncompressed to expand to a larger outer diameter.

11. The implantable prosthesis (100) according to any one of the preceding claims, in which the first framework has a first starting location generally diametrically opposed to a second starting location of the second framework as referenced with the longitudinal axis.

12. The implantable prosthesis (100) of any one of the preceding claims, in which the implantable prosthesis (100) comprises a biocompatible metal including alloys of metal.

13. The implantable prosthesis (100) of any one of the claims 1 to 11, in which the implantable prosthesis (100) comprises a biocompatible polymer.

14. The implantable prosthesis (100) of any one of the preceding claims, in which the implantable prosthesis (100) comprises a combination of biocompatible metal and polymer.

15. The implantable prosthesis (100) of any one of the preceding claims, in which the implantable prosthesis (100) comprises a drug coating disposed on surfaces of the implantable prosthesis.

## Patentansprüche

1. Implantierbare Prothese (100) umfassend:
a. ein erstes Gerüst (102), das einen ersten spiralförmigen Weg (101) um eine Längsachse (L-L) in der Nähe eines ersten Endes (100a) bis in die Nähe eines zweiten Endes (100b) der Prothese umschreibt, wobei das erste Gerüst eine Vielzahl von Streben (102a, 102b) umfasst, die in einem ersten sinusoiden Muster (103) dem ersten spiralförmigen Weg (101) entlang angeordnet sind, wobei ein primärer Spitzenteil (102c) für jedes Paar Streben (102a, 102b) angeschlossen ist;
b. ein zweites Gerüst (104) das von dem ersten Gerüst (102) beabstandet ist, wobei das zweite Gerüst einen zweiten spiralförmigen Weg (105) um die Längsachse (L-L) in der Nähe des ersten Endes (100a) bis in der Nähe des zweiten Endes (100b) umschreibt, wobei das zweite Gerüst (104) eine Vielzahl von Streben (104a, 104b) umfasst, die in einem zweiten sinusoiden Muster (107) dem zweiten spiralförmigen Weg (105) entlang angeordnet sind, wobei ein sekundärer Spitzenteil (104c) an ein Paar Streben für jedes Paar Streben (104a, 104b) angeschlossen; und
c. eine Vielzahl von Verbindungsstücken (106), wobei jedes Verbindungsstück (106) zum Annähern an einen primären Spitzenteil (102c) des ersten Gerüsts (102) und zum Annähern an einen sekundären Spitzenteil (104c) des zweiten Gerüsts (104) angeschlossen ist,
wobei der erste spiralförmige Weg (101) und der zweite spiralförmige Weg (105) verschachtelt sind und jeder primäre Spitzenteil (102c) mit einem sekundären Spitzenteil (104c) an einem benachbarten Gerüst angeschlossen ist und wobei jeder primäre Spitzenteil (102c) ein Vertex ist, der zirkumferentiell von jedem entsprechenden sekundären Gipfelteil (100 4c) versetzt ist.

2. Implantierbare Prothese (100) nach Anspruch 1, wobei jedes der Vielzahl von Verbindungsstücken (106) ein lineares Element umfasst, wobei wahlweise benachbarte Verbindungsstücke (106) in einer allgemein parallelen Konfiguration angeordnet sind.

3. Implantierbare Prothese (100) nach Anspruch 1, wobei die implantierbare Prothese (100) eine erste Länge (L1) umfasst, wenn sie gecrimpt oder komprimiert wird, um einen kleineren Außendurchmesser und eine zweite Länge von nicht weniger als etwa 80 % der ersten Länge aufzuweisen, wenn die implantierbare Prothese (100) nicht komprimiert ist, um sich zu einem größeren Außendurchmesser zu erweitern.

4. Implantierbare Prothese (100) nach einem der vorhergehenden Ansprüche, wobei das erste Gerüst einen ersten spiralförmigen Winkel (α1) mit Bezug auf die Längsachse (L-L) definiert und das zweite Gerüst einen zweiten spiralförmigen Winkel (α2) mit Bezug auf die Längsachse (L-L) definiert, wenn die implantierbare Prothese (100) in einer nicht komprimierten Konfiguration vorliegt.

5. Implantierbare Prothese (100) nach einem der vorhergehenden Ansprüche, wobei die Verbindungsstücke einen dritten spiralförmigen Winkel (α3) mit Bezug auf die Längsachse (L-L) definieren, wenn die implantierbare Prothese (100) in einer nicht komprimierten Konfiguration vorliegt.

6. Implantierbare Prothese (100) nach einem der vorhergehenden Ansprüche, wobei der erste spiralförmige Winkel (α1) allgemein dem zweiten spiralförmigen Winkel (α2) gleich ist.

7. Implantierbare Prothese (100) nach Anspruch 5 oder 6, wobei der dritte spiralförmige Winkel (α3) von dem ersten spiralförmigen Winkel (α1) oder dem zweiten spiralförmigen Winkel (α2) verschieden ist;
wobei wahlweise der dritte spiralförmige Winkel (α3) geringer ist als einer von dem ersten spiralförmigen Winkel (α1) oder dem zweiten spiralförmigen Winkel (α2).

8. Implantierbare Prothese (100) nach einem der vorhergehenden Ansprüche, wobei der erste spiralförmige Winkel (α1) etwa 65 Grad mit Bezug auf die Längsachse (L-L) beträgt; und/oder
wobei der zweite spiralförmige Winkel (α2) etwa 65 Grad mit Bezug auf die Längsachse (L-L) beträgt.

9. Implantierbare Prothese (100) nach einem der Ansprüche 5 bis 8, wobei der dritte spiralförmige Winkel (α3) etwa 30 Grad mit Bezug auf die Längsachse (L-L) beträgt; und/oder
wobei der dritte spiralförmige Winkel (α3) etwa die Hälfte von einem des ersten und des zweiten spiralförmigen Winkels (α1, α2) beträgt.

10. Implantierbare Prothese (100) nach einem der vorhergehenden Ansprüche, wobei die implantierbare Prothese (100) eine erste Länge, wenn sie gecrimpt oder komprimiert (L1) ist, um einen kleineren Außendurchmesser aufzuweisen, und eine zweite Länge von nicht weniger als etwa 90 % der ersten Länge umfasst, wenn die implantierbare Prothese (100) nicht komprimiert ist, um sich zu einem größeren Außendurchmesser zu erweitern.

11. Implantierbare Prothese (100) nach einem der vorhergehenden Ansprüche, wobei das erste Gerüst eine erste Ausgangsposition aufweist, die allgemein einer zweiten Ausgangsposition des zweiten Gerüsts, wie mit der Längsachse referenziert, diametral entgegengesetzt ist.

12. Implantierbare Prothese (100) nach einem der vorhergehenden Ansprüche, wobei die implantierbare Prothese (100) ein biologisch verträgliches Metall, einschließlich Metalllegierungen, umfasst.

13. Implantierbare Prothese (100) nach einem der Ansprüche 1 bis 11, wobei die implantierbare Prothese (100) ein biologisch verträgliches Polymer umfasst.

14. Implantierbare Prothese (100) nach einem der vorhergehenden Ansprüche, wobei die implantierbare Prothese (100) eine Kombination von biologisch verträglichem Metall und Polymer umfasst.

15. Implantierbare Prothese (100) nach einem der vorhergehenden Ansprüche, wobei die implantierbare Prothese (100) eine Medikamentbeschichtung umfasst, die auf Oberflächen der implantierbaren Prothese angeordnet ist.

## Revendications

1. Prothèse implantable (100) comprenant:
a. une première armature (102) de circonscription dans un premier trajet hélicoïdal (101) autour d'un axe longitudinal (L-L) proche d'une première extrémité (100a) pour s'approcher d'une seconde extrémité (100b) de la prothèse, la première armature comprenant une pluralité d'entretoises (102a, 102b) disposées selon un premier motif sinusoïdal (103) le long du premier trajet hélicoïdal (101) avec une partie d'apex primaire (102c) raccordée pour chaque paire d'entretoises (102a, 102b);
b. une seconde armature (104) espacée de la première armature (102), la seconde armature de circonscription d'un second trajet hélicoïdal (105) autour de l'axe longitudinal (L-L) proche de la première extrémité (100a) pour s'approcher de la seconde extrémité (100b), la seconde armature (104) comprenant une pluralité d'entretoises (104a, 104b) disposées selon un second motif sinusoïdal (107) le long du second trajet hélicoïdal (105) avec une partie d'apex secondaire (104c) raccordée à une paire d'entretoises pour chaque paire d'entretoises (104a, 104b); et
c. une pluralité de connecteurs (106), chaque connecteur (106) étant raccordé pour s'approcher d'une partie d'apex primaire (102c) de la première armature (102) et pour s'approcher d'une partie d'apex secondaire (104c) de l'armature secondaire (104),
dans laquelle le premier trajet hélicoïdal (101) et le second trajet hélicoïdal (105) sont intercalés; et, chaque partie d'apex primaire (102c) étant raccordée à une partie d'apex secondaire (104c) sur une armature adjacente et dans laquelle chaque partie d'apex primaire (102c) est un sommet décalé de manière circonférentielle de chaque partie d'apex secondaire (104c) correspondante.

2. Prothèse implantable (100) selon la revendication 1, dans laquelle chacun de la pluralité de connecteurs (106) comprend un élément linéaire, facultativement dans laquelle des connecteurs (106) adjacents sont disposés en une configuration généralement parallèle.

3. Prothèse implantable (100) selon la revendication 1, dans laquelle la prothèse implantable (100) comprend une première longueur (L1) lorsque plissée ou comprimée pour présenter un diamètre externe plus petit et une seconde longueur de pas moins d'environ 80 % de la première longueur lorsque la prothèse implantable (100) n'est pas compressée pour s'étendre jusqu'à un diamètre externe plus grand.

4. Prothèse implantable (100) selon l'une quelconque des revendications précédentes, dans laquelle la première armature définit un premier angle hélicoïdal (α1) par rapport à l'axe longitudinal (L-L) et la seconde armature définit un deuxième angle hélicoïdal (α2) par rapport à l'axe longitudinal (L-L) lorsque la prothèse implantable (100) se trouve dans une configuration non compressée.

5. Prothèse implantable (100) selon l'une quelconque des revendications précédentes, dans laquelle les connecteurs définissent un troisième angle hélicoïdal (α3) par rapport à l'axe longitudinal (L-L) lorsque la prothèse implantable (100) se trouve dans une configuration non compressée.

6. Prothèse implantable (100) selon l'une quelconque des revendications précédentes, dans laquelle le premier angle hélicoïdal (α1) est généralement égal au deuxième angle hélicoïdal (α2).

7. Prothèse implantable (100) selon la revendication 5 ou 6, dans laquelle le troisième angle hélicoïdal (α3) est différent du premier angle hélicoïdal (α1) ou du deuxième angle hélicoïdal (α2);
facultativement, dans laquelle le troisième angle hélicoïdal (α3) est inférieur soit au premier angle hélicoïdal (α1) soit au deuxième angle hélicoïdal (α2).

8. Prothèse implantable (100) selon l'une quelconque des revendications précédentes, dans laquelle le premier angle hélicoïdal (α1) est d'environ 65 degrés par rapport à l'axe longitudinal (L-L); et/ou,
dans laquelle le deuxième angle hélicoïdal (α2) est d'environ 65 degrés par rapport à l'axe longitudinal (L-L).

9. Prothèse implantable (100) selon l'une quelconque des revendications 5 à 8, dans laquelle le troisième angle hélicoïdal (α3) est d'environ 30 degrés par rapport à l'axe longitudinal (L-L); et/ou,
dans laquelle le troisième angle hélicoïdal (α3) est d'environ la moitié de l'un du premier et du deuxième angle hélicoïdal (α1, α2).

10. Prothèse implantable (100) selon l'une quelconque des revendications précédentes, dans laquelle la prothèse implantable (100) comprend une première longueur lorsque plissée ou comprimée (L1) pour présenter un diamètre externe plus petit et une seconde longueur de pas moins d'environ 90 % de la première longueur lorsque la prothèse implantable n'est compressée pour s'étendre jusqu'à un diamètre externe plus grand.

11. Prothèse implantable (100) selon l'une quelconque des revendications précédentes, dans laquelle la première armature présente une première localisation de départ généralement diamétralement opposée à une seconde localisation de départ de la seconde armature telle que référencée avec l'axe longitudinal.

12. Prothèse implantable (100) selon l'une quelconque des revendications précédentes, dans laquelle la prothèse implantable (100) comprend un métal biocompatible incluant des alliages de métal.

13. Prothèse implantable (100) selon l'une quelconque des revendications 1 à 11, dans laquelle la prothèse implantable (100) comprend un polymère biocompatible.

14. Prothèse implantable (100) selon l'une quelconque des revendications précédentes, dans laquelle la prothèse implantable (100) comprend une combinaison de métal et de polymère biocompatible.

15. Prothèse implantable (100) selon l'une quelconque des revendications précédentes, dans laquelle la prothèse implantable (100) comprend un revêtement de médicament disposé sur des surfaces de la prothèse implantable.
